# FASCICULE DE BREVET EUROPEEN

(11) **EP 0 844 879 B1**
(45) Date de publication et mention de la délivrance du brevet: **03.03.2004**
(21) Numéro de dépôt: 96916194.2
(22) Date de dépôt: 10.05.1996
(51) Int. Cl.: A61K 31/56, A61P 37/04

(54) **COMPOSITIONS PHARMACEUTIQUES CONTENANT DES DERIVES DE STEROIDES IMPLIQUES DANS LE DECLENCHEMENT DE LA REPONSE IMMUNITAIRE**
PHARMAZEUTISCHE ZUSAMMENSETZUNGEN VON STEROIDEN ZUR FÖRDERUNG DER IMMUNANTWORT
PHARMACEUTICAL COMPOSITIONS CONTAINING STEROID DERIVATIVES INVOLVED IN TRIGGERING AN IMMUNE RESPONSE

(30) Priorité: 11.05.1995 FR 9505607
(43) Date de publication de la demande: 03.06.1998
(73) Titulaire: LABORATOIRES MAYOLY SPINDLER, 78401 Chatou Cédex (FR)
(72) Inventeur: MORFIN, Robert, F-75013 Paris (FR); RONCO, Jorge, F-69350 La Mulatière (FR)
(74) Mandataire: Corizzi, Valérie
(86) Numéro de dépôt international: PCT/FR1996/000719
(87) Numéro de publication internationale: WO 1996/035428

(56) Documents cités:
- WO-A-94/03176
- WO-A-94/08588

## Description

La présente invention est relative à des compositions pharmaceutiques contenant à titre de principe actif des nouveaux dérivés de stéroïdes qui ont une fonction de restauration des mécanismes agissant sur la réponse immunitaire et plus particulièrement des mécanismes liés à l'immunité cellulaire B et T.

La formation des hormones stéroïdes, leurs interrelations et leurs fonctions ont été décrites dans un nombre très important d'articles et les fonctions plus précisément de la pregnènolone ou de la déhydroépiandrostérone (DHEA) ou de certains dérivés de la DHEA sont résumées dans l'état de la technique de la demande de brevet PCT WO 94/08588.

Le rôle physiologique des stéroïdes 7-hydroxylés tel la 7α-hydroxypregnenolone et les 7α/β-hydroxy-DHEA ont été partiellement élucidés par la démonstration de leur effet promoteur de l'immunité chez la souris (MORFIN et COURCHAY, J Steroïd. Biochem. Molec. Biol., 50 : 91-100, 1994) ; Dans le même ordre d'idée, la demande de brevet WO 93/20687 revendique le 5-DIOL et son dérivé 7β-hydroxylé comme agent régulateur de la réponse immunitaire. Cependant toutes ces molécules décrites présentent un certain nombre d'inconvénients qui sont notamment leur hydrosolubilité élevée, et une biodisponibilité insuffisante due à une fuite cellulaire trop rapide, ce qui en particulier présente un inconvénient majeur lorsque l'on souhaite utiliser cette capacité de restauration ou de stimulation de l'immunité cellulaire dans un processus de vaccination.

La présente invention est relative à l'utilisation dans la fabrication d'une composition stimulante de l'immunité, et en particulier pouvant conférer à un vaccin une immunité accrue, de composés répondant à l'une des trois formules générales suivantes : 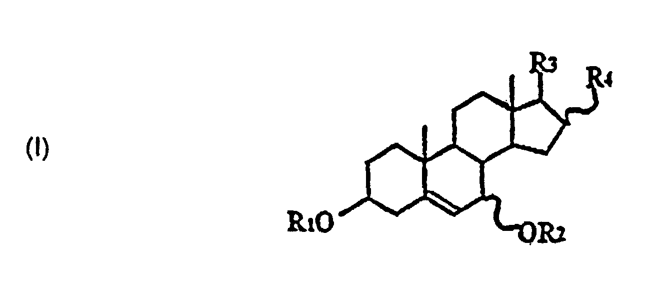 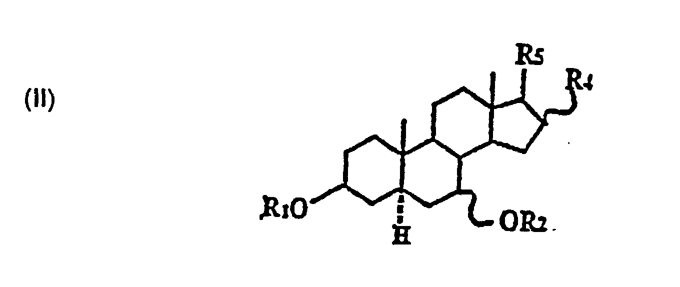 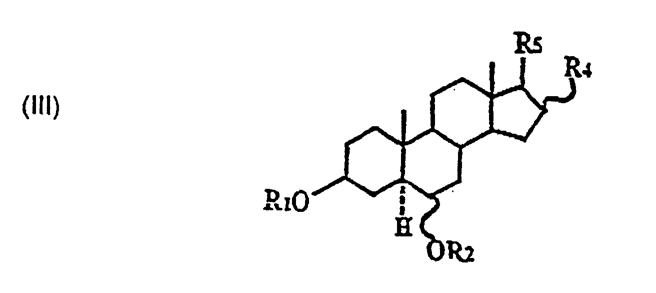 dans lesquelles :
R₁ représente H , un radical acyle R'₂ -CO- dans lequel R'₂ comprend de 1 à 25 atomes de carbone, un groupement SO₃H ou un groupement H₂PO₃,
R₂ représente H ou un radical acyle R'₂-CO- dans lequel R'₂ comprend de 1 à 25 atomes de carbone, R₁ étant différent de R₂,
R₃ étant choisi dans le groupe constitué de H, -CO-R_{6,} -CHOH-R_{6,} = O, le radical = 0 étant exclu quand R₁ est un hydrogène.
R₄ est H, ou un halogène ou un radical carbonitrile - C≡N,
R₅ est choisi dans le groupe constitué de H,-CO-R_{6,}-CHOH-R_{6,} = O, β-OH.
R₆ étant un groupe alcoyle comprenant de 1 à 10 atomes de carbone, substitué ou non, l'un au moins des groupements R₃ ou R₄ dans la formule I ou des groupements R₄ ou R₅ dans les formules II et III étant égal à H.

De façon particulière, les composés utilisés selon l'invention sont des dérivés 6α ou 7α substitués de la DHEA ou de la pregnenolone et plus particulièrement encore des dérivés 6α ou 7α hydroxylés réduits ou non réduits en position 5.

La composition stimulante de l'immunité comprenant un composé de formule (I) à (III) fait elle-même partie de l'invention, que le composé soit seul ou combiné à d'autres constituants, notamment à un antigène, ou à un autre adjuvant ou à une combinaison de ceux-ci ; en particulier, un vaccin contenant une composition de l'invention ou administré simultanément à ladite composition fait partie de l'invention, simultanément signifiant ici que les deux produits sont administrés à un patient dans la même forme galénique, ou dans deux formes galéniques distinctes et administrées dans un intervalle de temps autorisant un recouvrement partiel ou total des effets de chacun des produits.

Ce qui est surprenant dans les résultats obtenus grâce à ces vaccins est que les stéroïdes utilisés dans la composition ne sont pas des adjuvants dans le sens classique du terme : ils n'ont pas le pouvoir d'augmenter aussi considérablement la réponse immunitaire chez les souris saines adultes ou jeunes que les adjuvants classiques. Par contre les souris âgées souffrant d'une dérégulation du système immunitaire recouvrent une réponse immunitaire proche de la normale après traitement par les compositions de l'invention. Les effets de l'âge sur la réponse immunitaire sont actuellement connus au travers des travaux scientifiques publiés et concernant les domaines suivants :
a) Capacité proliférative affaiblie pour les cellules T âgées :
   lorsque les cellules présentatrices d'antigène présentent un antigène étranger à une cellule T, celle-ci est stimulée et produit de l'interleukine-2 (IL-2). Cette production d'IL-2 et la réponse à l'IL-2 sont diminuées dans les cellules T de personnes âgées. Par ailleurs, chez la souris âgée, on observe aussi une diminution de l'IL-3, du GM-CSF, de la réaction type DTH et de l'induction de CTL. Par contre, IL-4, IL-5, IL-6 et INFγ sont augmentés par rapport aux souris adultes jeunes (Weskler, Immune Senescence, Annals of NeuroL, suppl. 35; S35-S37 (1994)). Il a également été démontré que les cellules T de souris âgées proliféraient moins bien que celles des jeunes lorsque ces cellules sont stimulées par un anticorps anti-CD28 (Engwerda et al., J. Immunol. 153 : 3740-3747 (1994)). En effet, la CD28, qui est une protéine présente sur toutes les cellules T périphériques, est une molécule accessoire dans la présentation d'antigènes. Elle se lie à la molécule B7 (présente sur les cellules B activées, les macrophages, les cellules dentritiques, les cellules de Langerhans et les cellules T activées). Il semble que la stimulation de la CD28 pourrait induire la sécrétion d'IL-2. Chez les souris âgées, l'expression de la CD28 sur les cellules T CD4+, CD8+, CD44+^{lo} (population naïve) et CD44+^{hi} (population T mémoire) reste la même que sur les cellules T des jeunes. Cependant, la réponse proliférative de ces molécules vis à vis d'une stimulation avec un anticorps anti-CD28 est plus basse chez les souris âgées que chez les jeunes. Ceci suggère que le mécanisme de signalisation intracellulaire est défectueux chez les souris âgées. Par ailleurs, l'accumulation de cellules T mémoires et ne répondant pas aux stimuli pourrait être responsable de la carence immunitaire chez les personnes âgées (Flurkey et al., Eur. J. Immunol., 22 : 931 (1992)). L'ensemble de ces phénomènes suggère que le mécanisme de signalisation intracellulaire est défectueux chez les cellules âgées au travers d'effets directs sur la mémoire immunologique des compartiments B et T des populations âgées. Les stéroïdes hydroxylés de l'invention auraient une fonction de restauration de ces mécanismes.
b) Affaiblissement de la réponse B chez les personnes âgées :
   bien que la littérature reste contradictoire sur ce point, certaines études montrent que le taux d'anticorps neutralisants, produits par des personnes âgées à la suite de vaccinations par le vaccin antigrippal trivalent, reste équivalent à celui induit chez les jeunes (Powers and Belshe, J. Infect. Dis., 167: 584-592 (1993)). Cependant, le consensus général sur ce point est que les vaccins inactivés employés aujourd'hui sont moins efficaces chez les personnes âgées que chez les jeunes pour induire des anticorps neutralisants. Cette inefficacité augmente avec l'âge des patients.
c) Inefficacité des vaccins chez les personnes âgées de plus de 65 ans :
   après vaccination par le vaccin de la grippe, seulement près de 60% des personnes âgées saines répondent avec une immunité protectrice ; leur taux d'anticorps neutralisants et la réponse T cytotoxique restent faibles (Weskler, Immune Senescence, Ann. Neuro., Suppl. 35: S35-S37 (1994)).
d) Augmentation de l'expression des autoanticorps chez les individus âgés :
   chez la souris, il apparaît que les cellules B CD5(-) produisent des anticorps contre un antigène étranger, alors que les cellules B CD5(+) produisent des autoanticorps. Chez les souris âgées, la stimulation de CD5(-) par un antigène étranger est moins efficace que chez les souris jeunes. Par contre, l'antigène étranger stimule de manière égale les CD5 (+) chez les souris jeunes et âgées. La résultante est une production d'autoanticorps proportionnellement plus élevée chez les âgées que chez les jeunes (Weskler, Immune Senescence, Ann. Neuro., Suppl. 35: S35-S37 (1994)).

A partir de ces constatations diverses, il paraissait nécessaire de rechercher des traitements qui permettaient de combattre la carence immunitaire observée chez les populations âgées et de ramener la réponse immunitaire au niveau mesuré chez les jeunes. Les demandes de brevet WO 93/20696 et WO 94/08588 décrivaient un certain nombre de dérivés 7-hydroxylés naturels utilisables dans le déclenchement des processus immunitaires ; cependant ces dérivés présentaient un certain nombre d'inconvénients déjà décrits plus haut et qui en faisaient des candidats médiocres comme adjuvants de vaccination soit pour des raisons de toxicité, soit pour des raisons de bio-disponibilité non satisfaisante.

Les composés de l'invention répondant aux formules (I), (II) ou (III) déjà données ci-dessus permettent d'envisager leur utilisation dans des compositions pharmaceutiques ou des vaccins pour restaurer des déficiences immunitaires chez les sujets âgés. Il faut remarquer que les formules (II) ou (III) sont des composés réduits en position 5 ce qui présente plusieurs avantages : ils sont plus faciles à synthétiser, et donc moins onéreux et ils présentent en outre une activité plus importante que les dérivés non réduits. Les substitutions en R₁ par des acides gras leur confèrent enfin une liposolubilité accrue ce qui présente l'avantage d'améliorer la rétention de ces composés dans les cellules notamment au niveau de leurs membranes et par conséquent de prolonger leur activité et leur effet sur l'immunité cellulaire;

Des composés préférés de l'invention sont ceux dans lesquels le substituant R2 est un hydrogène autrement dit les dérivés 6 ou 7 hydroxylés. Des dérivés préférés de l'invention sont ceux où R₁ est un dérivé de l'acide oléique ou de l'acide palmitique, et notamment le 3β palmitoyl 7α - OH-DHEA.
Les composés des formules I, II ou III peuvent être obtenus par synthèse chimique classique, selon des méthodes connues de l'homme du métier.

Les compositions ci-dessus peuvent en outre contenir un principe actif de vaccin.

La présente invention est également relative à des compositions vaccinales ou des vaccins comprenant outre le principe actif au moins un adjuvant constitué d'un des dérivés décrits ci-dessus.

La dose d'adjuvant efficace est située entre 0,05 et 10mg par kg de poids corporel et par dose, et de préférence entre 0,05 et 5 mg.

L'immunisation par administration de vaccin est réalisée soit par le mélange de l'antigène avec l'adjuvant dans la composition elle-même, soit l'immunisation est réalisée par administration simultanée de l'antigène et d'un adjuvant constitué d'au moins un composé de l'invention, « simultanée » ayant la même signification que ci-dessus. L'adjuvant dans les vaccins ou compositions vaccinales de l'invention est constitué d'au moins un dérivé des stéroïdes selon l'invention, associé le cas échéant, avec un autre dérivé décrit par exemple dans WO 93/20687 ou WO 94/08588, ou encore un adjuvant de type alum, le dérivé de l'invention en tout état de cause étant présent à la dose de 0,05 à 10 mg par kg et de préférence entre 0,05 et 5 mg par kg.

Cet effet de restauration ou de stimulation de l'immunité notamment chez les personnes d'un certain âge peut être appliqué à tout traitement visant à restaurer l'immunité cellulaire B et T, et notamment dans les cas de cancers et de maladies autoimmunes.

La nature même des dérivés de l'invention fait que les formes galéniques envisageables et leurs voies d'administration sont larges. Les voies d'administration peuvent être outre les voies classiques intramusculaires, sous-cutanées, intra-dermiques, intra-veineuses, intra-vaginales, intra-rectales, il est possible d'envisager des formes orales, intra-péritonéales, intra-puimonaires, mucco-nasales ou transcutanées. Les formes galéniques correspondantes sont des formes résultant de l'addition aux principes actifs et aux adjuvants de composés acceptables pharmaceutiquement et permettant de réaliser des crèmes, des pâtes, des émulsions « eau dans huile » ou « huile dans eau », ainsi que des formes composées de liposomes de micelles simples ou mixtes ou autre promoteur de pénétration tels des lyso-phospholipides, des cyclodextrines, du PEG (polyéthylène glycol), des tensioactifs, du DMSO, de l'acétone, des alcools, des acides gras, des huiles végétales ; d'autres formes galéniques envisageables sont des formes de type retard ou permettant un relargage contrôlé du principe actif.

Des pilules ou capsules administrables par voie orale ou des implants sont d'autres formes galéniques utilisables pour les composés de l'invention. Cette liste de formes galéniques n'est pas non plus exhaustive dans la mesure où d'autres formes peuvent être mises au point et tout à fait adaptables aux dérivés stéroïdiens de l'invention qui en outre ont comme caractéristiques essentielles d'être à la fois liposolubles et hydrosolubles.

Fait également partie de l'invention l'utilisation d'un stéroïde de l'invention dans la fabrication d'un vaccin ou une utilisation dans la fabrication d'une composition pharmaceutique destinée à compenser les effets du vieillissement sur la réponse immunitaire. Dans cette utilisation, le dérivé de stéroïde est administré à une dose comprise entre 0,05 mg/kg et 10 mg/kg et de préférence entre 0,05 et 5 mg/kg, la composition pharmaceutique étant un vaccin humain ou vétérinaire.

Quand il s'agit d'un vaccin humain, cela peut notamment être un vaccin contre la grippe, ou un vaccin contre l'hépatite.
Les exemples ci-dessous montrent les performances obtenues par les dérivés de l'invention comme activateurs de l'immunité et comme adjuvants de vaccins.

### Exemple 1 : Utilisation du 3β-palmitoyl -7αOH-DHEA en tant qu'adjuvant de vaccin :

Un protocole d'immunisation contre le vaccin monovalent de la grippe (vaccin NIB16, souche A) (HA), est utilisé pour tester l'effet adjuvant du dérivé 3β-palmitoyl de la 7αOH-DHEA mis en solution à 10 mg/ml dans le DMSO.

Trois lots de 5 souris BALB/C âgées de 6 semaines (jeunes) et trois lots de 5 souris BALB/C âgées de 90 semaines (âgées) subissent le premier jour une injection sous-cutanée de 0,5 ml de sérum physiologique contenant :
soit 8 µg de HA + 1,8 µmole (0,78 mg/kg) de 3β-palmitoyl-7αOH-DHEA (lot 1)
soit 8 µg de HA + 1,8 µmole (0,56 mg/kg) de 7αOH-DHEA (lot 2)
soit 8 µg de HA (lot 3)
Après 28 jours, chaque souris de chaque lot subit un rappel, les injections décrites étant répétées pour chaque lot. Après 38 jours, chaque souris de chaque lot est sacrifiée et le sang prélevé.
Les IgG totales anti-HA sont mesurées dans chaque sérum par une méthode immunoenzymatique. Les résultats sont indiqués dans le tableau 1 ci-dessous où l'effet du 3β-palmitoyl-7αOH-DHEA rétablit la réponse immunitaire des souris âgées à un niveau proche de celui des souris jeunes.

**Tableau 1**

| Lot | Traitement | Souris jeunes | Souris âgées |
|---|---|---|---|
| 1 | HA + 3β-palmitoyl-7αOH-DHEA | 0,70 ± 0,47 | 0,69 ± 0,48 |
| 2 | HA +7αOH-DHEA | 1,34 ± 0,35 | 0,21 ± 0,09 |
| 3 | HA | 0,56 ± 0.30 | 0,27 ± 0,16 |

### Exemple 2 : Utilisation de la 7αOH-DHEA et de son dérivé 3β-palmitoyl en tant qu'adjuvants complémentaires de l'Alum :

Un protocole d'immunisation contre le virus monovalent de la grippe (souche Taiwan A86) (HA) est utilisé pour tester l'effet adjuvant de la 7αOH-DHEA et du HA + 3β-palmitoyl-7αOH-DHEA en complément de celui connu pour l'Alum.
Deux lots de 5 souris BALB/C âgées de 6 semaines (jeunes) et trois lots de 5 souris BALB/C âgées de 90 semaines (âgées) subissent le premier jour une injection sous-cutanée de 0,5 ml de sérum physiologique contenant, pour les deux lots « jeunes » :
soit 5 µg HA
soit 5 µg HA + 55,5 mg/kg Alum
et pour les trois lots « âgées »,
soit 5 µg HA
soit 5 µg HA + 55,5 mg/kg Alum,
soit 5 µg HA + 55,5 mg/kg Alum + 50 µg (2,8 mg/kg) 7αOH-DHEA + 50µg (2,8 mg/kg) 3β-palmitoyl-7αOH-DHEA.
Après 28 jours (réponse immunitaire), chaque souris subit un prélèvement sanguin par la voie exolacrimale, suivi d'un rappel, les injections décrites étant répétées pour chaque lot. Après 38 jours (réponse secondaire), chaque souris est sacrifiée et le sang prélevé.
Les réponses primaires et secondaires correspondent aux titre anti-HA de chaque sérum et sont mesurées pour les IgGI par la méthode immunoenzymatique.
Les résultats du tableau 2 montrent que le traitement des souris âgées par le mélange Alum+ 3β-palmitoyl-7αOH-DHEA + 7αOH-DHEA aux doses indiquées confère aux souris âgées une protection anti-HA significativement supérieure à celle induite par les traitements sans stéroïde.

**Tableau 2**

| Traitement | Réponse primaire, jeunes | Réponse primaire, âgées | Réponse secondaire, jeunes | Réponse secondaire, âgées |
|---|---|---|---|---|
| HA | 1515 ± 687 | 127 ± 111 | 4905 ± 2704 | 768 ± 448 |
| HA + Alum | 2440 ± 1556 | 153 ± 140 | 8052 ± 4518 | 1749 ± 1656 |
| HA + Alum + 3β-palmitoyl-7αOH-DHEA +7αOH-DHEA | - | 1435 ± 1419 | | 3811 ± 2395 |

Les résultats présentés dans les exemples ci-dessus montrent clairement et notamment à la lecture des tableaux 1 et 2 que l'addition de dérivés 3β-palmitoyl-7αOH-DHEA a un effet sur la réponse immunitaire des souris âgées comparable à celui obtenu chez les souris jeunes ; la comparaison notamment avec les dérivés 7αOH-DHEA seuls pour lequel cette réponse n'est pas obtenue montre l'effet inattendu et extrêmement prometteur comme adjuvant de vaccin des dérivés de l'invention.

## Revendications

1. Composition pour utilisation dans un médicament stimulant de l'immunité ou dans un vaccin **caractérisée en ce qu'**elle contient un composé répondant à l'une des formules suivantes : 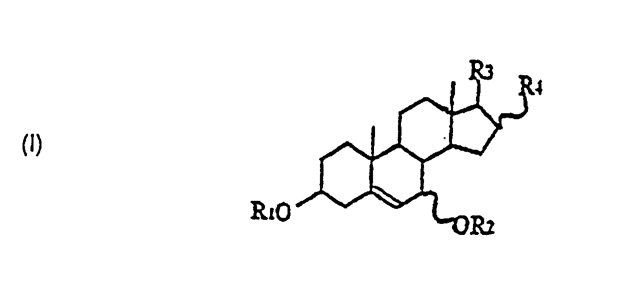 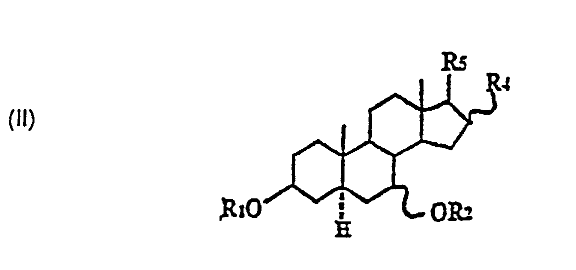 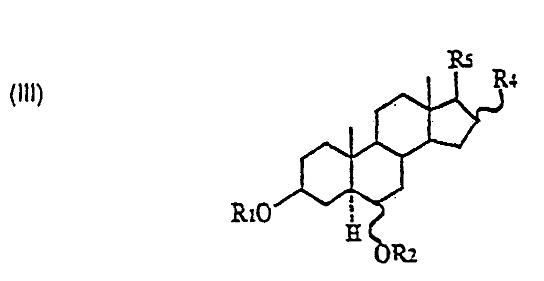 dans lesquelles : R₁ représente H, un radical acyle R'₂-CO- dans lequel R'₂ comprend de 1 à 25 atomes de carbone, un groupement SO₃H ou un groupement H₂PO₃, R₂ représente H ou un radical acyle R'₂-CO- dans lequel R'₂ comprend de 1 à 25 atomes de carbone, R₁ étant différent de R₂,
R₃ étant choisi dans le groupe constitué de H, -CO-R₆, -CHOH-R₆, =O, le radical =O étant exclu quand R₁ est un hydrogène.
R₄ est H, ou un halogène ou un radical carbonitrile -C≡N,
R₅ est choisi dans le groupe constitué de H, -CO-R₆, -CHOH-R₆, =O, β-OH.
R₆ étant un groupe alcoyle comprenant de 1 à 10 atomes de carbone, substitué ou non, l'un au moins des groupements R₃ ou R₄ dans la formule (I) ou des groupements R₄ ou R₅ dans les formules (II) et (III) étant égal à H.

2. Composition selon la revendication 1 **caractérisée en ce que** R₁ est un dérivé de l'acide oléique ou de l'acide palmitique.

3. Composition selon la revendication 1 ou 2 **caractérisée en ce que** R₂ est un hydrogène.

4. Composition selon l'une des revendications 1 à 3 **caractérisée en ce que** R₃ et R₅ représentent le groupement CH₃-CO-.

5. Composition selon l'une des revendications 1 à 3 **caractérisée en ce qu'**elle contient du 3β palmitoyl-7α OH-DHEA.

6. Utilisation d'une composition selon l'une quelconque des revendications 1 à 5 pour la fabrication d'un vaccin.

7. Utilisation d'une composition selon l'une quelconque des revendications 1 à 5 pour la fabrication d'un médicament destiné à être administré simultanément à un vaccin.

8. Utilisation d'une composition selon une des revendications 1 à 5 pour la fabrication d'un médicament destiné à compenser les effets du vieillissement sur la réponse immunitaire.

## Patentansprüche

1. Zusammensetzung zur Verwendung in einem immunstimulierenden Medikament oder in einem Impfstoff, **dadurch gekennzeichnet, daß** sie eine Verbindung gemäß einer der folgenden Formeln enthält: 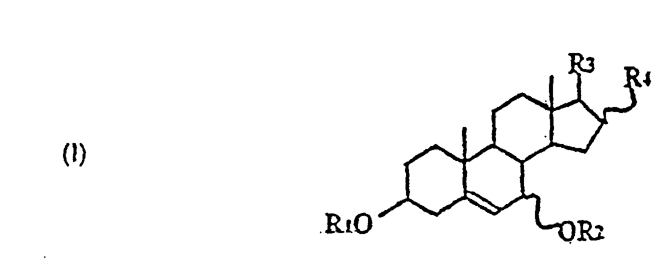 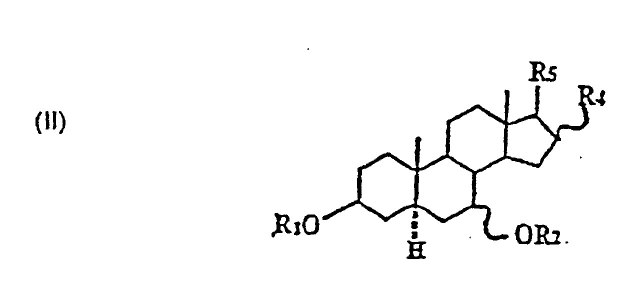 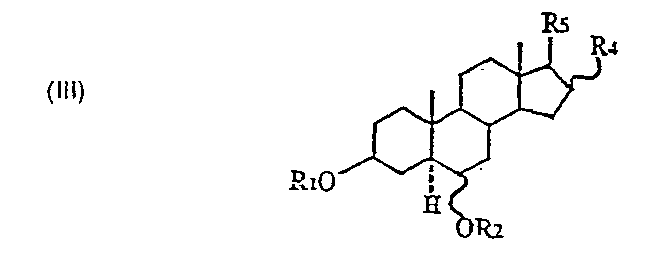 in welchen: R₁ bedeutet H, einen Acylrest R'₂-CO-, worin R'₂ 1 bis 25 Kohlenstoffatome umfaßt, eine SO₃H-Gruppe oder eine H₂PO₃-Gruppe, R₂ bedeutet H oder einen Acylrest R'₂-CO-, worin R'₂ 1 bis 25 Kohlenstoffatome umfaßt, wobei R₁ von R₂ verschieden ist,
R₃ ist aus der Gruppe ausgewählt bestehend aus H, -CO-R₆, -CHOH-R₆, =O, wobei der Rest =O ausgenommen ist, wenn R₁ Wasserstoff ist,
R₄ ist H oder ein Halogen oder ein Carbonitrilrest - C=N,
R₅ ist ausgewählt aus der Gruppe bestehend aus H, -CO-R₆, -CHOH-R₆, =O, β-OH,
wobei R₆ eine substituierte oder unsubstituierte Alkylgruppe ist, die 1 bis 10 Kohlenstoffatome umfaßt, wobei wenigstens eine der Gruppen R₃ oder R₄ in der Formel (I) oder der Gruppen R₄ oder R₅ in den Formeln (II) und (III) H ist.

2. Zusammensetzung nach Anspruch 1, **dadurch gekennzeichnet, daß** R₁ ein Oleinsäure- oder Palmitinsäurederivat ist.

3. Zusammensetzung nach Anspruch 1 oder 2, **dadurch gekennzeichnet, daß** R₂ Wasserstoff ist.

4. Zusammensetzung nach einem der Ansprüche 1 bis 3, **dadurch gekennzeichnet, daß** R₃ und R₅ die Gruppe CH₃-CO- bedeuten.

5. Zusammensetzung nach einem der Ansprüche 1 bis 3, **dadurch gekennzeichnet, daß** sie 3β Palmitoyl-7α OH-DHEA enthält.

6. Verwendung einer Zusammensetzung nach irgendeinem der Ansprüche 1 bis 5 zur Herstellung eines Impfstoffs.

7. Verwendung einer Zusammensetzung nach irgendeinem der Ansprüche 1 bis 5 zur Herstellung eines Medikaments zur gleichzeitigen Verabreichung mit einem Impfstoff.

8. Verwendung einer Zusammensetzung nach einem der Ansprüche 1 bis 5 zur Herstellung eines Medikaments zur Kompensation der Wirkungen des Alterns auf die Immunreaktion.

## Claims

1. Composition for use in an immunity-stimulating medication or in a vaccine, **characterised in that** it contains a compound complying with one of the following formulae: 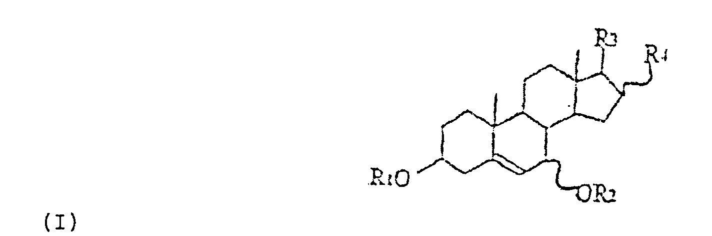 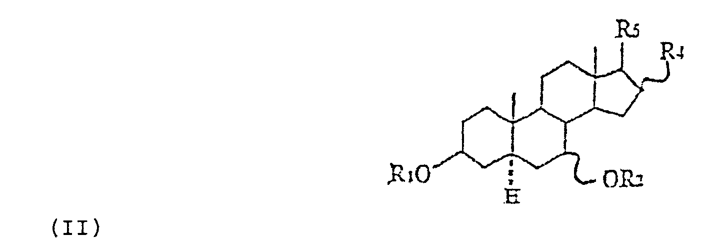 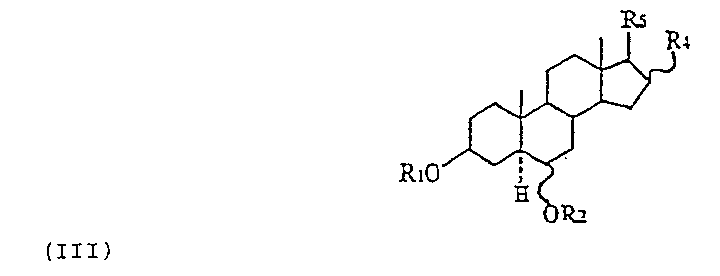 in which: R₁ represents H, an alkyl radical R'₂-CO- in which R'₂ comprises 1 to 25 carbon atoms, an SO₃H group or an H₂PO₃ group, R₂ represents H or an alkyl radical R'₂-CO- in which R'₂ comprises 1 to 25 carbon atoms, R₁ being different from R₂,
R₃ being chosen from the group consisting of H, -CO-R₆, -CHOH-R₆, =O, the radical =O being excluded when R₁ is hydrogen,
R₄ is H, or a halogen or a carbonitrile radical -C≡N,
R₅ is chosen from the group consisting of H, -CO-R₆, -CHOH-R₆, =O, β-OH,
R₆ being an alkyl group comprising 1 to 10 carbon atoms, substituted or not, at least one of the groups R₃ or R₄ in formula (I) or groups R₄ or R₅ in formulae (II) and (III) being equal to H.

2. Composition according to Claim 1, **characterised in that** R₁ is a derivative of oleic acid or palmitic acid.

3. Composition according to Claim 1 or 2, **characterised in that** R₂ is hydrogen.

4. Composition according to one of Claims 1 to 3, **characterised in that** R₃ and R₅ represent the group CH₃-CO-.

5. Composition according to one of Claims 1 to 3,
**characterised in that** it contains 3β palmitoyl-7α OH-DHEA.

6. Use of a composition according to any one of Claims 1 to 5 for manufacturing a vaccine.

7. Use of a composition according to any one of Claims 1 to 5 for manufacturing a medication intended to be administered simultaneously with a vaccine.

8. Use of a composition according to one of Claims 1 to 5 for manufacturing a medication intended to compensate for the effects of aging on the immune response.
